(19) Europäisches Patentamt
European Patent Office
Office européen des brevets

(11) EP 1 029 841 B1

(12) EUROPEAN PATENT SPECIFICATION

(45) Date of publication and mention of the grant of the patent:
**09.04.2003 Bulletin 2003/15**

(51) Int Cl.[7]: **C07C 33/025**, C11B 9/00, A61K 7/46

(21) Application number: **00102807.5**

(22) Date of filing: **11.02.2000**

# (54) 6-Substituted 3-methyloct-6-enols

6-Substituierte 3-Methyloct-6-enole

3-Méthyloct-6-énols substitués en position 6

(84) Designated Contracting States:
**AT BE CH CY DE DK ES FI FR GB GR IE IT LI LU MC NL PT SE**

(30) Priority: **19.02.1999 EP 99103243**

(43) Date of publication of application:
**23.08.2000 Bulletin 2000/34**

(73) Proprietor: **Givaudan SA**
**1214 Vernier-Genève (CH)**

(72) Inventors:
- **Frater, Georg**
  **8400 Winterthur (CH)**
- **Kraft, Philip**
  **8600 Dübendorf (CH)**
- **Mueller, Urs**
  **8600 Dübendorf (CH)**

(74) Representative: **Patentanwälte**
**Schaad, Balass, Menzl & Partner AG**
**Dufourstrasse 101**
**Postfach**
**8034 Zürich (CH)**

(56) References cited:
**FR-A- 338 895** **FR-A- 1 562 796**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 029 841 B1

## Description

The invention is concerned with mixtures of the (6*E*)- and (6*Z*)-isomers of 3,6-dimethyloct-6-en-1-ols and 6-ethyl-3-methyloct-6-en-1-ols and their mixtures as well as the use of these mixtures in odorant compositions. The mixtures are free from their corresponding oct-5-ene double bond isomers.

Although it has previously not been known that one compound alone can imitate true to nature the complex olfactory impression of lily of the valley, hydroxycitronellal (3,7-dimethyl-7-hydroxyoctan-1-al) comes remarkably close to the odour of lily of the valley. In combination with other lily of the valley odorants such as Lyral® [4-(4-hydroxy-4-methylpent-1-yl)-cyclohex-3-ene-1-carboxaldehyde], Lilial® [3-(4-tert-butylphenyl)-2-methylpropanal] and/or Dupical® [4-(octahydro-4,7-methano-5H-inden-5-ylidene)butanal] (G. Fráter, J.A. Bajgrowicz, P. Kraft, Fragrance Chemistry, Tetrahedron 1998, 54, 7633-7703) it is, however, possible to come very close to the natural standard. The aldehyde group is common to all of these mentioned odorants with lily of the valley character. This gives rise to an instability of the compounds in oxidizing or strongly alkaline media.

Some lily of the valley odorants without an aldehyde function have been found in the alcohols Mayol® (4-(1-methylethyl)cyclohexylmethanol), Majantol® (2,2-dimethyl-3-(3-methylphenyl)propanol), Florol® (tetrahydro-4-methyl-2-(2-methylpropyl-2H-pyran-4-ol) and Mugetanol® [U. Harder, E. Oelkers, in: Recent Developments in Flavor and Fragrance Chemistry, published by VCH, Weinheim, 1993, pp. 162-163]. Moreover, the carbon analogue to Florol with very similar olfactory properties to Florol® has become known from WO 98/47842. However, these alcohols possess neither the naturalness, radiating strength and crispness nor the olfactory strength of the aforementioned aldehydes, and the need for a replacement substance for the lily of the valley aldehydes therefore continues to exist. There is therefore a requirement for additional lily of the valley odorants, especially with a functional group other than the aldehyde function, in order to obviate the previously mentioned disadvantages.

In GB Patent 1,167,776 claim is made, inter alia, to a supposed compound of general formulae Ia and Ib.

From the examples given therein it is, however, evident that compounds falling within formulae **Ia** and **Ib** were never prepared in pure form. It has now been established that this is also not at all possible on the basis of the route of preparation described. In fact, in the dehydration of the 1,6-diols which are the basis for the compounds **Ia** and **Ib** there is obtained a mixture of all three possible isomeric octan-1-ols with respect to the double bond. For example, in the case of compounds **Ib** there are obtained according to the given process the alcohols (*6E*)-6-ethyl-3-methyloct-6-en-1-ol (*ca.* 30%), (6*Z*)-6-ethyl-3-methyloct-6-en-1-ol (*ca.* 30%) and 6-ethyl-3-methyloct-5-en-1-ol (*ca.* 40%). As mentioned in the patent, this mixture of compounds **Ib** has, in fact, the indicated odour after lily of the valley and rose, but the odour of the rose side notes is unpleasantly musty. Because of this musty rose-like side note the mixture **Ib** can not completely satisfy the requirements of a lily of the valley odorant without an aldehyde function. It has therefore been used in perfumery only sporadically. Today there is practically no longer any commercial demand. The production of the mixture **Ib** has in the meanwhile been discontinued for this reason.

It has now surprisingly been found that the undesired musty rose-like side notes of the mixtures **Ia** and **Ib** is due to the oct-5-en-1-ols 6-ethyl-3-methyloct-5-en-1-ol and (5*E*/*Z*)-3,6-dimethyloct-5-en-1-ol and that in the absence of these compounds there are obtained mixtures which do not have the aforementioned disadvantages. The object of the invention are therefore the mixtures of general formula II

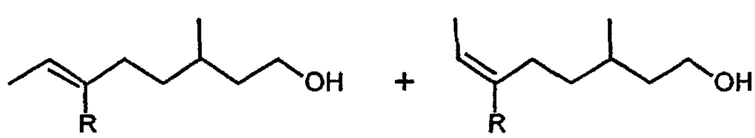

II

including all R- and S-enantiomers, in which R can stand for a methyl or ethyl group. The class of compound accordingly embraces the two mixtures $II_1$ and $II_2$ and their mixtures.

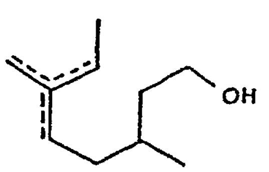

Ia

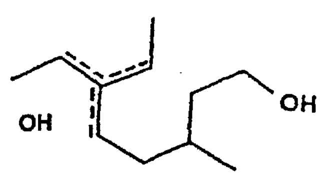

Ib

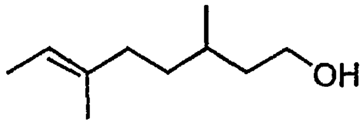 + 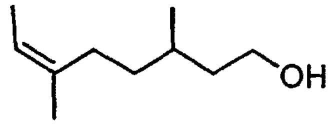

II$_1$

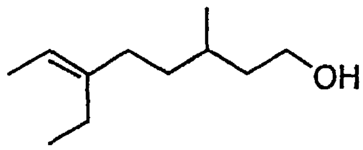 + 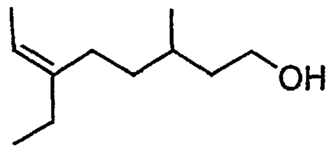

II$_2$

The mixture II$_1$ has a floral, crisp-aldehydic odour typical of lily of the valley with surprisingly a delicate citric nuance and a threshold value of 10 ng/l air. More surprising, however, is the fact that the mixture II$_2$ even has an extremely uniform and independent, specific lily of the valley fragrance, floral, crisp-aldehydic, with an even somewhat lower threshold value than mixture II$_1$, namely of 6 ng/l air. The musty-rose like aspects of the mixtures **Ia** and **Ib** are no longer present; on the contrary new, independent fragrance notes can now be created. The mixtures II$_1$ and II$_2$ are therefore ideally suited - unlike the mixtures **Ia** and **Ib** - as lily of the valley odorants without an aldehyde function, which surpass the known aldehyde substitutes in radiating strength, crispness and olfactory uniformity. The citric olfactory nuance of the mixture II$_1$ is a surprising new occurrence. It is presumed that this was therefore not previously recognized in the mixtures **Ia** and **Ib** according to GB Patent 1,167,776, because it was masked by the musty rose-like note.

The mixtures II$_1$ and II$_2$ or their mixtures are thus outstandingly suitable for perfumes of the crisp-flowery type, especially of the so-called Eaux Fraiches, such as, for example, Eau d'Eden (Cacharel, 1996), Eau d'Issey (I. Miyake, 1997), Eau belle (L. Azzaro, 1995), Eternity (C. Klein, 1988), Escape (C. Klein, 1991), New West for her (Aramis, 1990), and particularly those with an accentuated lily of the valley character, such as, for example, Diorissimo (Dior, 1956), Pleasures (E. Lauder, 1995), Aqua di Gio (Armani, 1994), Hugo Woman (H. Boss, 1997), Envy (Gucci, 1996), Polo Sport Woman (R. Lauren, 1996).

The use is, however, limited neither to these perfume types, nor to special olfactory directions, odorants or classes of substance. The following are set forth as examples of classes of substance which harmonize especially well with the mixtures in accordance with the invention.

| | |
|---|---|
| - Ethereal oils and extracts, e.g. | bergamot oil, cedarwood oil, galbanum oil, jasmin absolute, rose oil, ylang-ylang oil. |
| -Alcohols, e.g. | citronellol, Dimetol®, dimethylphenylethyl carbinol, Ebanol®, ethyllinalool, geraniol, Peonil®, phenylethyl alcohol, Radjanol®, Undecavertol®. |
| - Aldehydes and ketones, e.g. | Adoxal®, alpha-damascone, Dupical®, Florhydral®, Hedione®, hydroxycitronellal, cis-jasmone, Lilial®, Lyral®, 4-(4-methoxyphenyl)-butan-2-one, Myraldene®, Nectaryl®, Scentenal®, Tricyclal®, Tropional®, Vertofix®. |
| - Ethers and acetals, e.g. | Acetal CD®, Ambrofix®, Calone®, diphenyl oxide, Folenox®, Galaxolide®, Glycolierral®, Limettol®, Magnolan®, Rhubafuran®, Spirambrene®. |
| - Esters and lactones, e.g. | Agrumex®, benzyl acetate, benzyl salicylate, citronellyl acetate, Gardenol®, cis-3-hexenyl salicylate, Myraldylacetate®, Prunolide®, cis-jasmone lactone, Jasmonyl®, gamma-undecalactone. |
| - Macrocycles, e.g. | Ambrettolide®, Ambretone®, Ethylenebrassylate®, Habanolide®, Muscone®, Musk CPD®, Musk 174®, Thibetolide®. |
| - Heterocycles, e.g. | indole, Pyralone®. |

The mixtures $II_1$ and $II_2$ were produced by the reaction of the Grignard reagent of THP-protected 5-bromo-3-methylpentan-1-ol with acetaldehyde or propionaldehyde, subsequent Dess-Martin oxidation, followed by a Wittig reaction with ethyltriphenylphosphonium bromide and acid-catalyzed deprotection. Other protecting groups (e.g. tert-butyldimethylsilyl) or oxidizing agents (e.g. pyridinium chlorochromate) can, however, also be employed. This approach, which yields the compounds free from the corresponding oct-5-ene double bond isomers, is illustrated in the following Schemes:

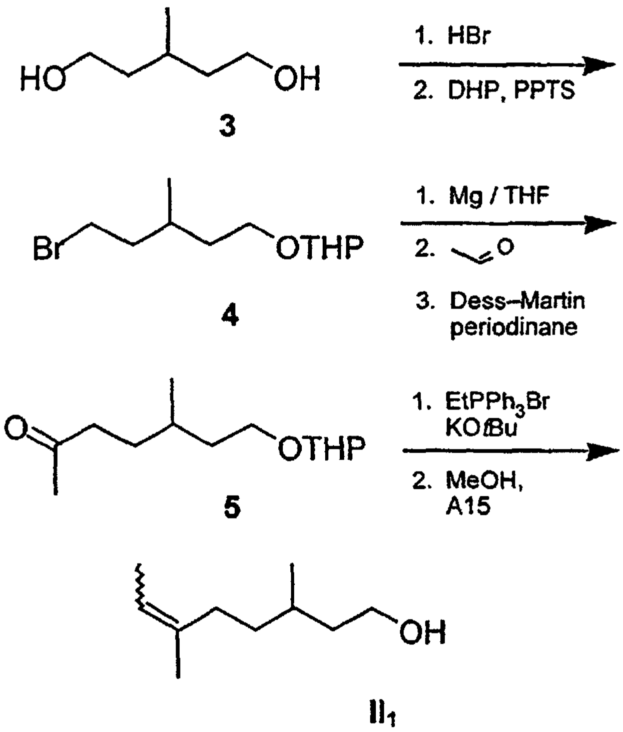


3

4

5

$II_1$

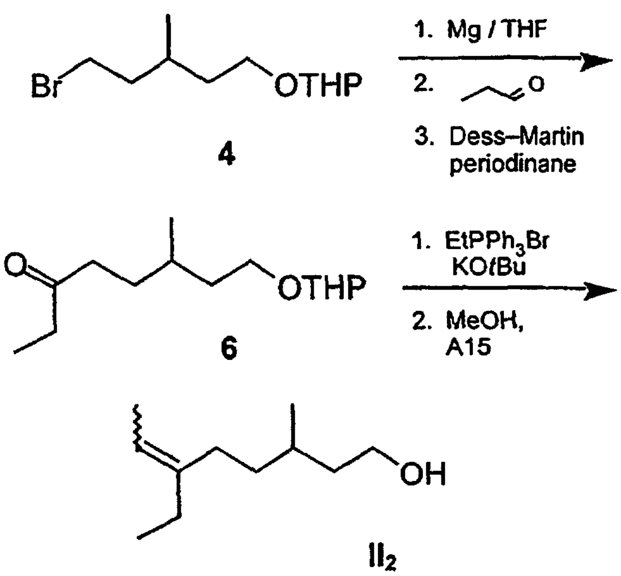


4

6

$II_2$

Further advantages, features and details will be evident from the following description of the Examples for

the production of the mixtures and of preferred working Examples with respect to the use of the mixtures.

**Example 1**

**(6E/Z)-3,6-Dimethyloct-6 -en-1-ol ($II_1$)**

A solution of 182 ml (1.50 mol) of3-methylpentane-1,5-diol (3) in 3 l of toluene was treated portionwise with a total of 185 ml (1.6 mol) of 48 percent hydrobromic acid and then heated to reflux (internal temperature 100-110°C) on a water separator for 5 hours. After 170 ml of water had been separated the reaction mixture was left to cool, poured on to 400 g of ice and treated with 500 ml of water and 30 ml of 30 percent sodium hydroxide solution. The organic phase was separated and washed with 750 ml of 2N hydrochloric acid, twice with 750 ml of water each time and then with 750 ml of saturated sodium chloride solution. After drying over sodium sulphate and concentration on a rotary evaporator there were obtained, after flash chromatography (*tert*-butyl methyl ether:*n*-pentane, 1:1, $R_f$ = 0.57) on silica gel, 106 g (39%) of 5-bromo-3-methylpentan-1-ol. A solution of 1.48 g (0.82 mol) of 5-bromo-3-methylpentan-1-ol from several analogous batches in 11 of dry dichloromethane was treated under nitrogen and while stirring and cooling in an ice-water bath with a solution of 107 g (1.27 mol) of 3,4-dihydropyran and 17.4 g (69.3 mmol) of pyridinium toluene-4-sulphonate in 1 of dry dichloromethane. After removal of the cooling the reaction mixture was left to stir at room temperature for a further 8 hours, then poured into 6 of water and the product was extracted twice with 500 ml of *tert*-butyl methyl ether each time. The combined organic extracts were dried over sodium sulphate and freed from solvent on a rotary evaporator. Flash chromatography (*n*-pentane:*tert*-butyl methyl ether, 20:1) on silica gel yielded 197 g (91%) of 5'-bromo-3'-methylpent-1'-yl tetrahydropyran-2-yl ether (4).

IR (film): ν = 1035/1077/1121/1135 $cm^{-1}$ (ν C-O), 1353/1381 $cm^{-1}$ (ν $CH_3$), 1454/1441 $cm^{-1}$ (ν $CH_2$).- $^1$H-NMR ($CDCl_3$): δ = 0.94 (d, *J* = 6.4 Hz, 3H, 3'-$H_3$), 1.43-1.71 (m, 8H, 3-,4-,5-, 2'-$H_2$), 1.82 ($m_c$, 2H, 3'-H, 4'-$H_b$), 1.93 ($m_c$, 1H, 4'-$H_a$), 3.37-3.52 (m, 4H, 1'-,5'-$H_2$), 3.77-3.87 (m, 2H, 6-$H_2$), 4.57 ($m_c$, 1H, 2-H). - $^{13}$C-NMR ($CDCl_3$): δ = 18.83/18.92 (2q, 3'-Me), 19.48 (2t, C-4), 25.35 (2t, C-5), 28.88/28.94 (2d, C-3'), 30.61 (2t, C-3), 31.67 (2t, C-5'), 35.94/36.03 (2t, C-2'), 39.82/39.93 (2t, C-4'), 62.15/62.18 (2t, C-1'), 65.16/65.34 (2t, C-6), 98.62/98.83 (2d, C-2). - MS (EI): *m*/*z* (%) = 41 (30) [$C_3H_5^+$], 55 (63) [$C_4H_7^+$], 85 (100) [$C_5H_9O^+$], 101 (3) [$C_5H_9O_2^+$, complementary to *m*/*z* = 163/165], 163/165 (9) [$C_6H_{12}Br^+$, complementary to *m*/*z* = 101], 263/265 (2) [$M^+$-H].

About 5 ml of a solution of 50.0 g (200 mmol) of 5'-bromo-3'-methylpent-1'-yl tetrahydropyran-2-yl ether (4) in 250 ml of dry tetrahydrofuran were added to 5.50 g (226 mmol) of magnesium shavings in 40 ml of dry tetrahydrofuran and the mixture was heated while stirring slowly with a KPG stirrer until the reaction began. Thereupon, the heating source was removed and the remainder of the 5'-bromo-3'-methylpent-1'-yl tetrahydropyran-2-yl ether solution was slowly added dropwise. Subsequently, the mixture was heated under reflux for 20 hours and, after cooling, a solution of 11.0 g (250 mmol) of acetaldehyde in 70 ml of dry tetrahydrofuran was slowly added dropwise. After stirring for 3 hours the reaction mixture was added to 1 of saturated ammonium chloride solution, the organic phase was separated and the aqueous phase was extracted twice with 300 ml of *tert*-butyl methyl ether each time. The combined organic phases were washed twice with 300 ml of saturated sodium chloride solution each time, dried over sodium sulphate and concentrated to dryness on a rotary evaporator. By flash chromatography (*n*-pentane:*tert*-butyl methyl ether, 5:1, $R_f$= 0.30) of the residue on silica gel there were obtained 26.5 g (58%) of 5'-methyl-2'-hydroxyhept-7'-yl tetrahydropyran-2-yl ether. This was taken up in 350 ml of dry dichloromethane and treated at room temperature with a solution of 73.1 g (173 mmol) of Dess-Martin periodinate in 350 ml of dry dichloromethane. After stirring at room temperature for 2 hours the reaction mixture was treated with 1 of *tert*-butyl methyl ether and a solution of 225 g (1.42 mmol) of sodium thiosulphate in 1 of saturated sodium hydrogen carbonate solution. After stirring at room temperature for 10 minutes the organic phase was separated, the aqueous phase was extracted twice with 500 ml of *tert*-butyl methyl ether each time and the combined organic phases were washed with saturated sodium hydrogen carbonate solution and saturated sodium chloride solution. After drying over sodium sulphate and removal of the solvent on a rotary evaporator there are obtained by flash chromatography (*n*-pentane:*tert*-butyl methyl ether, 10:1, $R_f$ = 0.42) on silica gel 23.1 g (88%) of 5'-methyl-2'-oxohept-7'-yl tetrahydropyran-2-yl ether (5).

IR (film): ν = 1717 $cm^{-1}$ (ν C=O), 1035/1078/1122/1136 $cm^{-1}$ (ν C-O), 1355 $cm^{-1}$ (ν $CH_3$), 1454/1441 $cm^{-1}$ (ν $CH_2$).- $^1$H-NMR ($CDCl_3$): δ = 0.91 (d, J = 5.6 Hz, 3H, 5'-$H_3$), 1.43-1.81 (m, 11H, 3-$H_2$-5-$H_2$ and 4'-$H_2$-6'-$H_2$), 2.15 (s, 3H, 1'-$H_3$), 2.42-2.48 (m, 2H, 3'-$H_2$), 3.38-3.53 (m, 2H, 7'-$H_2$), 3.74-3.88 (m, 2H, 6-$H_2$), 4.56/4.57 (2t, *J* = 4.2/4.0 Hz, 1H, 2-H). - $^{13}$C-NMR ($CDCl_3$): δ = 19.17/19.28 (2q, C-1'), 19.47/19.52 (2q, 5'-Me), 25.27 (2t, C-5), 29.37/29.41 (2d, C-5'), 30.48/30.57 (2t, C-3), 36.18/36.22 (2t, C-6'), 41.14/41.11 (2t, C-3'), 62.14/62.21 (2t, C-7'), 65.32/65.51 (2t, C-6), 98.61/98.94 (2d, C-2), 208.97/209.60 (2s, C-2'). - MS (EI): *m*/*z* (%) = 43 (48) [$C_3H_7^+$], 55 (15) [$C_4H_7^+$], 69 (27) [$C_5H_9^+$], 85 (100) [$C_5H_9O^+$], 101 (18) [$C_8H_{15}O^+$, complementary to *m*/*z* = 127], 109 (43) [$C_8H_{13}^+$], 127 (35) [$M^+$-$C_8H_{15}O$, complementary to m/z = 101], 143 (8) [$M^+$-$C_5H_9O$], 227 (1) [$M^+$-H].

14.7 g (39.5 mmol) of ethyltriphenylphosphonium bromide were added under nitrogen to a solution of 4.25 g (37.8 mmol) of potassium *tert*-butylate in 50 ml of dry tetrahydrofuran. The reaction mixture was heated to reflux and

at this temperature a solution of 7.50 g (32.9 mmol) of 5'-methyl-2'-oxohept-7'-yl tetrahydropyran-2-yl ether (5) in 25 ml of dry tetrahydrofuran was allowed to drop in. After stirring under reflux for 2 hours and at room temperature for 8 hours the reaction mixture was added to 400 ml of *tert*-butyl methyl ether/water (1:1), the organic phase was separated and the aqueous phase was extracted three times with 100 ml of *tert*-butyl methyl ether each time. The organic phases were combined, dried over sodium sulphate and concentrated on a rotary evaporator. By flash chromatography (*n*-pentane:*tert*-butyl methyl ether, 100:1, $R_f$ = 0.44) on silica gel there were obtained 7.15 g (90%) of 3',6'-dimethyloct-2'-en-8'-yl tetrahydropyran-2-yl ether as a colourless liquid. A solution of 7.00 g (29.1 mmol) of the 3',6'-dimethyloct-2'-en-8'-yl tetrahydropyran-2-yl ether in 200 ml of dry methanol was treated with 10 g of Amberlyst® 15 and stirred for 20 hours at room temperature. The ion exchanger was filtered off and extracted twice with 100 ml of methanol. After concentration of the combined organic phases on a rotary evaporator there were obtained by flash chromatography (*n*-pentane:*tert*-butyl methyl ether, 10:1, $R_f$= 0.14) 3.86 g (85%) of (6*E*/*Z*)-3,6-dimethyloct-6-en-1-ol ($II_1$) as a colourless liquid with an intensive odour.

Odour: Floral, crisp-aldehydic, after lily of the valley with a light citrus nuance. - IR (film): ν = 3338 $cm^{-1}$ (ν O-H), 1059 $cm^{-1}$ (ν C-O), 1457 $cm^{-1}$ (ν $CH_2$), 1378 $cm^{-1}$ (ν $CH_3$). - $^1$H-NMR ($CDl_3$): δ = 0.89/0.90 (2d, *J* = 6.8/6.4 Hz, 3H, 3-Me), 1.26-1.18 (m, 1H, 3-H), 1.35-1.43 (m, 2H, 4-$H_2$), 1.52-1.66 (m, 2H, 2-$H_2$), therein 1.56 (d, *J* = 6.8 Hz, 3H, 8-$H_3$), 1.59/1.67 (2s, 3H, 6-Me), 1.98-2.04 (m, 2H, 5-$H_2$), 2.42 (br s, 1H, OH), 3.61-3.70 (m, 2H, 1-$H_2$), 5.19 ($m_c$, 1H, 7-H). - $^{13}$C-NMR ($CDCl_3$): δ = 13.03/13.20 (2q, C-8), 19.44 (2q, 3-Me), 15.48/23.27 (2q, 6-Me), 29.12/29.47 (2d, C-3), 28.70/35.03/35.33/36.93 (4t, C-4,-5), 39.68/39.71 (2t, C-2), 60.79 (2t, C-1),117.93/118.51 (d, C-7), 136.24/135.96 (2s, C-6). - MS (EI): *m*/*z* (%) = 41 (100) [$C_3H_5^+$], 55 (88) [$C_4H_7^+$], 70 (73) [$C_5H_{10}^+$], 81 (35) [$C_6H_9^+$], 109 (19) [$C_8H_{13}^+$], 123 (4) [$M^+$-$H_2O$-$CH_3$],138 (2) [$M^+$+CHO], 156 (8) [$M^+$].

**Example 2**

**(6E/Z) -6-Ethyl-3-methyloct-6-en-1-ol ($II_2$)**

The corresponding Grignard reagent was prepared in analogy to Example 1 from 1.65 g (67.8 mmol) of magnesium shavings and 18.9 g (67.8 mmol) of 5'-bromo-3'-methylpent-1'-yl tetrahydropyran-2-yl ether (4) in 85 ml of dry tetrahydrofuran. After heating under reflux for 3 hours the reaction mixture was left to cool to 30°C and treated dropwise with 4.32 g (74.6 mmol) of propionaldehyde dissolved in 30 ml of dry tetrahydrofuran. After the exothermic reaction had subsided the mixture was stirred at room temperature for a further 1 hour and thereupon added to 500 ml of saturated ammonium chloride solution. The organic phase was separated and the aqueous phase was extracted three times with 100 ml of *tert*-butyl methyl ether each time. The combined organic phases were washed twice with 150 ml of saturated sodium chloride solution each time and then dried over sodium sulphate. After removal of the solvent on a rotary evaporator there were obtained by flash chromatography (*n*-pentane:*tert*-butyl methyl ether, 5:1, $R_f$= 0.30) of the residue on silica gel 5.87 g (36%) of 6'-methyl-3'-hydroxyoct-8'-yl tetrahydropyran-2-yl ether. This was dissolved in 75 ml of dry dichloromethane and treated at room temperature with 15.3 g (36.0 mmol) of Dess-Martin periodinate while stirring vigorously. After stirring for 2 hours 250 ml of *tert*-butyl methyl ether were added, followed by a solution of 48 g of sodium thiosulphate in 250 ml of saturated aqueous sodium hydrogen carbonate solution. After stirring for 10 minutes the organic phase was separated, the aqueous phase was extracted twice with 200 ml of *tert*-butyl methyl ether and the combined organic extracts were washed with saturated sodium hydrogen carbonate solution and saturated sodium chloride solution. After drying over sodium sulphate and removal of the solvent on a rotary evaporator there were obtained by flash chromatography (*n*-pentane:*tert*-butyl methyl ether, 10:1, $R_f$ = 0.31) 5.00 g (94%) of 6'-methyl-3'-oxooct-8'-yl tetrahydropyran-2-yl ether (6) as a colourless oil.

IR (film): ν = 1716 $cm^{-1}$ (ν C=O), 1034/1078/1122/1136 $cm^{-1}$ (ν C-O), 1353/1378 $cm^{-1}$ (ν $CH_3$), 1456 $cm^{-1}$ (ν $CH_2$). - $^1$H-NMR ($CDl_3$): δ = 0.91 (d, *J* = 6.0 Hz, 3H, 6'-$H_3$), 1.05 (t, *J* = 7.4 Hz, 3H, 1'-$H_3$), 1.41-1.81 (m, 11H, 3-$H_2$-5-$H_2$ and 5'-$H_2$-7'-$H_2$), 2.37-2.48 (m, 4H, 2'-,4'-$H_2$), 3.36-3.53 (m, 2H, 8'-$H_2$), 3.74-3.88 (m, 2H, 6-$H_2$), 4.56/4.57 (2t, J = 3.5/4.0 Hz, 1H, 2-H). - $^{13}$C-NMR ($CDCl_3$): δ = 7.65 (2q, C-1'), 19.19/19.30 (2q, 6'-Me), 19.48/19.52 (2t, C-4), 25.28 (2t, C-5), 29.44/29.49 (2d, C-6'), 30.57/30.57/30.60/30.67 (4t, C-3,-5'), 35.62/35.64/36.19/ 36.24 (4t, C-2'-7'), 39.75/39.77 (2t, C-4'), 62.14/62.21 (2t, C-8'), 65.36/65.54 (2t, C-6), 98.62/98.84 (2d, C-2), 211.58/211.59 (2s, C-3'). - MS (EI): *m*/*z* (%) = 57 (53) [$C_4H_5$], 85 (100) [$C_5H_9O$], 101 (10) [$C_5H_8O$], 123 (19) [$C_8H_{11}O$], 141 (32) [$M^+$-$C_5H_9O_2$], 158 (8) [$M^+$-$C_5H_8O$], 213 (1) [$M^+$-$C_2H_5$], 241 (1) [$M^+$-H].

Likewise in analogy to Example 1, 9.10 g (24.6 mmol) of ethyltriphenylphosphonium bromide were added under nitrogen to a solution of 2.60 g (23.2 mmol) of potassium *tert*-butylate in 40 ml of dry tetrahydrofuran. The reaction mixture was heated to reflux and at this temperature a solution of 9.10 g (24.6 mmol) of 6'-methyl-3'-oxooct-8'-yl tetrahydropyran-2-yl ether (6) in 10 ml of dry tetrahydrofuran was allowed to drop in. After stirring under reflux for 33 hours and at room temperature for 8 hours the reaction mixture was added to 300 ml of *tert*-butyl methyl ether/water (1:1), the organic phase was separated and the aqueous phase was extracted three times with 100 ml of *tert*-butyl methyl ether each time. The organic phases were combined, dried over sodium sulphate and concentrated on a rotary

evaporator. After flash chromatography (*n*-pentane:*tert*-butyl methyl ether, 100:1, $R_f$ = 0.39) on silica gel there were obtained 4.62 g (96%) of 3'-ethyl-6'-methyloct-2'-en-8'-yl tetrahydropyran-2-yl ether as a colourless liquid. A solution of 4.50 g (18.9 mmol) of the 3'-ethyl-6'-methyloct-2'-en-8'-yl tetrahydropyran-2-yl ether in 150 ml of dry methanol was treated with 7.50 g of Amberlyst® 15 and stirred for 16 hours at room temperature. The ion exchanger was filtered off and extracted twice with 150 ml of methanol. After concentration of the combined organic phases on a rotary evaporator there were obtained by flash chromatography (*n*-pentane:*tert*-butyl methyl ether, 10:1, $R_f$ = 0.22) 2.52 g (78%) of (6*E*/*Z*)-6-ethyl-3-methyloct-6-en-1-ol ($II_2$) as a colourless liquid with an intensive odour.

Odour: Extremely uniform and independent, specifically after lily of the valley, floral, crisp-aldehydic. - IR (film): $\nu$ = 3328 $cm^{-1}$ ($\nu$ O-H), 1058 $cm^{-1}$ ($\nu$ C-O), 1459 $cm^{-1}$ ($\nu$ $CH_2$), 1377 $cm^{-1}$ ($\nu$ $CH_3$). - $^1$H-NMR ($CDCl_3$): $\delta$ = 0.90-0.99 (m, 6H, 2'-$H_3$, 3-Me), 1.18-1.26 (m, 1H, 3-H), 1.35-1.45 (m, 2H, 4-$H_2$), 1.51-1.66 (m, 2H, 2-$H_2$), therein 1.57 (d, *J* = 6.8 Hz, 3H, 8-$H_3$), 1.96-2.06 (m, 4H, 5-,1'-$H_2$), 2.15 (br s, 1H, OH), 3.60-3.71 (m, 2H, 1-$H_2$), 5.17 ($m_c$, 1H, 7-H). - $^{13}$C-NMR ($CDCl_3$): $\delta$ = 12.73/12.74/12.86/12.98 (4q, C-8, C-2'), 19.45/19.48 (2q, 3-Me), 22.62/27.17 (2t, C-1'), 29.27/29.67 (2d, C-3), 29.57/33.87 (2t, C-5), 35.48/35.61 (2t, C-4), 39.69/39.76 (2t, C-2), 60.90 (2t, C-1), 116.78/117.47 (2d, C-7), 141.97 (2s, C-6). - MS (EI): *m*/*z* (%) = 31 (8) [$CH_2OH^+$], 41 (38) [$C_3H_5^+$], 55 (85) [$C_4H_7^+$], 69 (84) [$C_5H_9^+$], 84 (100) [$C_6H_{12}^+$], 97 (19) [$C_7H_{13}^+$], 123 (18) [$M^+$-$H_2O$-$C_2H_5$], 141 (5) [$M^+$-CHO], 170 (21) [$M^+$].

The compounds II are ideally suited for the creation of crisp-flowery lily of the valley accords, as is demonstrated hereinafter in Example 3. The mixtures II, especially the mixture $II_1$, intensify the floral base accord.

The compounds II, especially the mixture $II_1$, is likewise ideally suited for use in cosmetic articles and body care agents, especially shower gels and foam baths, for the emphasis of a floral, crisp-aldehydic specific olfactory impression of lily of the valley, whereby the odorant mixture in accordance with the invention does not lead to skin irritations or discoloration. On the other hand, the lily of the valley aldehydic odorants of the state of the art also frequently cause discolorations in addition to skin irritations.

## Example 3

### Unisex Eau Fraîche

Unisex Eau Fraîche with hesperidic top notes, a clean-flowery middle note with lily of the valley, ionone and jasmin bouquet as well as green, crisp-marine accents and an amber like-woody base note with reminiscences of iris and sandalwood:

| Compound/Ingredient | | Parts by weight in %o |
|---|---|---|
| 1. | Allylcyclohexylglycolate, 10% in dipropylene glycol | 20 |
| 2. | Bergamot oil | 150 |
| 3. | 3-Butyltetrahydro-5-methyl-2H-pyran-4-yl acetate [Jasmonyl®] | 50 |
| 4. | Lemon oil, Italian | 25 |
| 5. | 2,6-Dimethylheptan-2-ol [Dimetol®] | 35 |
| 6. | 3,7-Dimethylnona-1,6-dien-3-ol [ethyl linalool] | 50 |
| 7. | Dipropylene glycol | 60 |
| 8. | Florhydral®, 10% in dipropylene glycol | 5 |
| 9. | 1,3,4,6,7,8-Hexahydro-4,6,6,7,8,8-hexamethyl-cyclopenta[g]-2-benzopyran [Galaxolide®] 50 PHT | 100 |
| 10. | *cis* -Hex-3-en-1-ol, 10% in dipropylene glycol | 5 |
| 11. | beta-Ionone | 35 |
| 12. | Spearmint oil, USA, 10% in dipropylene glycol | 5 |
| 13. | Mandarin oil, Italian | 10 |
| 14. | 7-Methyl-2H-1,5-benzodioxepin-3(4H)-one [Calone 1951®], 10% in dipropylene glycol | 5 |
| 15. | Methyl dihydrojasmonate | 200 |
| 16. | 1-Methyl-3-[4-(1,1-dimethylethyl)phenyl] propanal [Lilial®] | 35 |
| 17. | 3-Methyl-5-(2,2,3-trimethyl-3-cyclopenten-1-yl)-4-penten-2-ol [Ebanol®], 10% in dipropylene glycol | 30 |
| 18. | 1-(1,2,3,4,5,6,7,8-Octahydro-1,2,8,8-tetramethyl-2-naphthalenylethanone [Georgywood®] | 30 |

(continued)

| | Compound/Ingredient | Parts by weight in %o |
|---|---|---|
| 19. | 3a,6,6,9a-Teramethyldodecahydro[2,1b]naphthofuran [Fixambrene®], 10% in dipropylene glycol | 75 |
| 20. | Tropional | 25 |
| 21. | Mixture **II** $_1$ | 50 |
| | | 1 000 |

The floral base accord is intensified by the mixture $II_1$. The mixture confers to it a crisp, intensive lily of the valley note without musty side aspects as, for example, when the mixture Ia or Ib is used. Thereby, the transparent, tea-like facets of the composition come into play especially well.

**Example 4**

**Crisp-flowery perfume composition for use in cosmetic articles and body care agents**

| Compound/Ingredient | Parts by weight in 1/1250 |
|---|---|
| 1. Ethyl acetoacetate | 25 |
| 2. Allylcyclohexylglycolate | 2 |
| 3. Benzyl acetate, purest | 35 |
| 4. Cassis Base 345 F | 5 |
| 5. Cedryl acetate | 20 |
| 6. Citronellyl acetate, 10% in dipropylene glycol | 5 |
| 7. Cyclopentadecanolide [Thibetolide®] | 5 |
| 8. 2,4-Dimethyl-3-cyclohexenecarboxaldehyde [Tricyclal®] | 4 |
| 9. 2,6-Dimethylhept-5-enal [Melonal®], 10% in dipropylene glycol | 7 |
| 10. 3,7-Dimethyloct-6-en-1-ol [Rhodinol®] | 35 |
| 11. Dipropylene glycol | 446.5 |
| 12. Dodecahydro-3a,6,6,9a-tetramethylnaphtho[2,1b]-furan, 10% in dipropylene glycol | 2 |
| 13. 3,7-Dimethylnona-1,6-dien-3-ol [ethyl linalool] | 50 |
| 14. 1,4-Dioxacycloheptadecane-5,17-dione [ethylene brassylate] | 5 |
| 15. 4-Ethyl-3-(4-ethylphenyl)-2,2-dimethylpropanal [Floralozone®] | 2 |
| 16. 1,3,4,6,7,8-Hexahydro-4,6,6,7,8,8-hexamethylcyclopenta[g]-2-benzopyran [Galaxolide®] 50 BB | 150 |
| 17. Hexyl salicylate | 5 |
| 18. alpha-Hexylcinnamaldehyde | 100 |
| 19. 4-(4-Hydroxy-4-methylpentyl)cyclohex-3-ene-1-carboxaldehyde [Cyclohexal®] | 25 |
| 20. Indole, pure, 10% in dipropylene glycol | 3 |
| 21. *cis*-Jasmone | 0.5 |
| 22. Linalool, synthetic | 8 |
| 23. Methyl dihydrojasmonate | 50 |
| 24. 1-Methyl-3-[4-(1,1-dimethylethyl)phenyllpropanal [Lilial®] | 80 |
| 25. 8-Methyl-beta-ionone [Isoraldeine®] | 35 |
| 26. 4-Methyl-2-(2-methylpropyl)tetrahydropyran-4-ol [Florol®] | 5 |
| 27. (4-Methylphenyl)acetaldehyde [Syringa aldehyde®], 10% in diethyl phthalate | 15 |
| 28. 3-Methyl-5-phenylpentan-1-ol [Phenoxanol®] | 10 |
| 29. Nerol, extra | 15 |
| 30. 1-(1,23,4,5,6,7,8-Octahydro-2,3,8,8-tetramethyl-2-naphthalenyl)ethanone [Iso E super®] | 20 |

(continued)

| Compound/Ingredient | Parts by weight in 1/1250 |
|---|---|
| 31. (10*E*)-1-Oxacycloheptadec-10-en-2-one [Ambrettolide®] | 10.5 |
| 32. 1-Phenyleth-1-yl acetate [Gardenol®] | 2.5 |
| 33. Phenylethyl alcohol | 20 |
| 34. Pepper oil, black | 2 |
| 35. Rose TW 62/9 Norx | 20 |
| 36. alpha-Terpineol [Lindenol®] | 5 |
| 37. 10-Undecen-1-al, 1% in dipropylene glycol | 10 |
| 38. Mixture $II_2$ | 10 |
| | 1250 |

The light, clear, crisp lily of the valley character of the mixture $II_2$ clearly comes into play in the composition, surprisingly even in this relatively low dosage, brings elegance and crispness into the olfactory picture and underlines the caring character of the product. The mixture $II_2$ can even intensify the effect of other alcohols with less pronounced lily of the valley odour, as shown here by the example of Florol®. Neither Majantol® nor Majol® or Mugetamol® is able to produce in this composition in like dosages a similar natural lily of the valley note, and the mixtures Ia and Ib are also not capable of this.

## Claims

1. **1.** Mixtures of general formula II

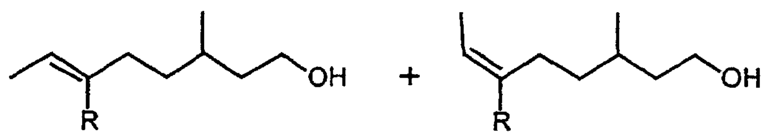

II

including all R- and S-enantiomers, wherein R represents methyl or ethyl, which are free from the corresponding oct-5-ene double bond isomers.

2. **2.** The mixture according to claim 1 containing (*6E*)-3,6-dimethyloct-6-en-1-ol and (6*Z*)-3,6-dimethyloct-6-en-1-ol ($II_1$), which is free from (5*E*/*Z*)-3,6-dimethyloct-5-en-1-ol.

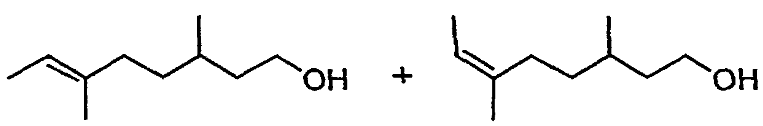

$II_1$

3. **3.** The mixture according to claim 1 containing (*6E*)-6-ethyl-3-methyloct-6-en-1-ol and (*6Z*)-6-ethyl-3-methyloct-6-en-1-ol ($II_2$), which is free from (5*E*/*Z*)-ethyl-3-methyloct-5-en-1-ol.

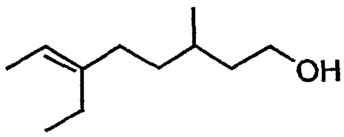 + 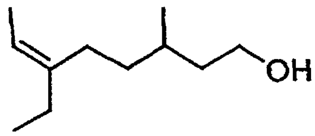

$II_2$

4. An odorant composition, which contains a mixture in accordance with any one of claims 1 to 3.

5. The use of the mixture in accordance with any one of claims 1 to 3 as an odorant.

**Patentansprüche**

1. Mischungen der allgemeinen Formel II

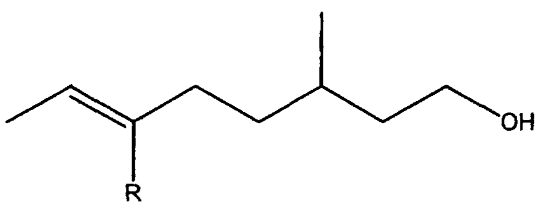 + 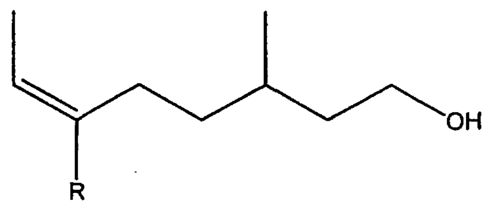 (II)

umfassend alle R- und S-Enantiomere, in der R Methyl oder Ethyl darstellt, und die frei von dem entsprechenden Oct-5-en Doppelbindungsisomeren sind.

2. Die Mischung nach Anspruch 1 enthaltend (6E)-3,6-Dimethyloct-6-en-1-ol und (6Z)-3,6-Dimethyloct-6-en-1-ol ($II_1$), die frei von (5E/Z)-3,6-Dimethyloct-5-en-1-ol ist.

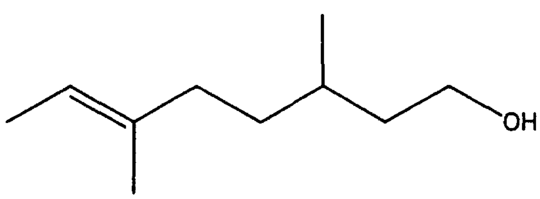 + 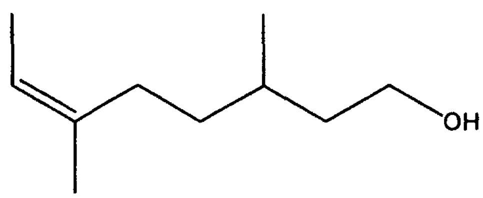 ($II_1$)

3. Die Mischung nach Anspruch 1, enthaltend (6E)-6-Ethyl-3-methyloct-6-en-1-ol und (6Z)-6-Ethyl-3-methyloct-6-en-1-ol ($II_1$), die frei von (5E/Z)-Ethyl-3-ethyloct-5-en-1-ol ist.

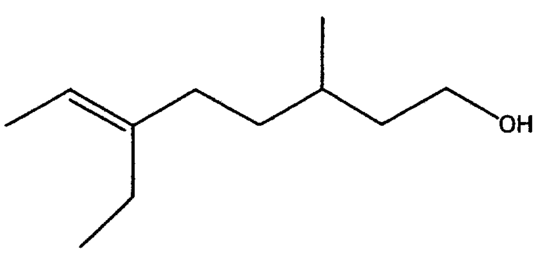 + 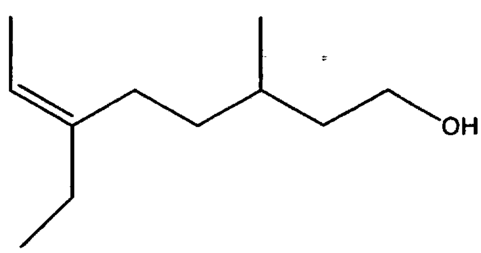 ($II_2$)

4. Eine duftende Zusammensetzung, die eine Mischung nach einem der Ansprüche 1 bis 3 enthält.

5. Verwendung der Mischung nach einem der Ansprüche 1 bis 3 als Duftstoff.

**Revendications**

1. Mélanges de formule générale II

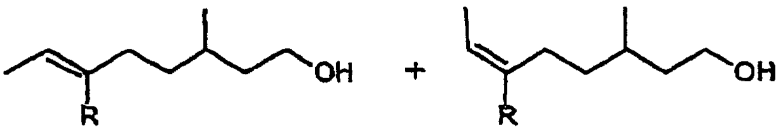

II

comprenant tous les énantiomères R- et S-, dans lesquels R représente un groupe méthyle ou éthyle, qui sont exempts des isomères à double liaison oct-5-ène correspondants.

2. Mélange selon la revendication 1 contenant du (6*E*)-3,6-diméthyloct-6 -èn-1-ol et du (6*Z*)-3,6-diméthyloct-6-èn-1-ol ($II_1$), qui est exempt de (5*E*/*Z*)-3,6-diméthyloct-5-èn-1-ol.

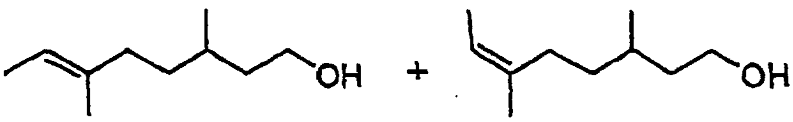

$II_1$

3. Mélange selon la revendication 1 contenant du (6*E*)-6-éthyl-3-méthyloct-6-èn-1-ol et du (6*Z*)-6-éthyl-3-méthyloct-6-èn-1-ol ($II_2$), qui est exempt de (5*E*/*Z*)-éthyl-3-méthyloct-5-èn-1-ol.

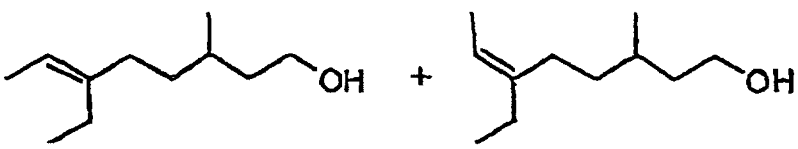

$II_2$

4. Composition odorante, qui contient un mélange selon l'une quelconque des revendications 1 à 3.

5. Utilisation du mélange selon l'une quelconque des revendications 1 à 3 comme substance odorante.